# EUROPEAN PATENT APPLICATION

(11) **EP 4 621 060 A1**
(43) Date of publication of application: **24.09.2025**
(21) Application number: 24799863.6
(22) Date of filing: 21.04.2024
(51) Int. Cl.: C12P 19/14, C12P 19/02, D21B 1/06, D21C 1/00

(54) **METHOD FOR IMPROVING ENZYMATIC HYDROLYSIS EFFICIENCY OF LIGNOCELLULOSE**

(30) Priority: 30.04.2023 CN 202310486936
(71) Applicant: Zhejiang Huakang Pharmaceutical Co., Ltd., Quzhou, Zhejiang 324302 (CN)
(72) Inventor: LI, Mian, Santa Clara, CA (US); YANG, Wulong, Quzhou, Zhejiang 324302 (CN); CHEN, Tianying, Quzhou, Zhejiang 324302 (CN); YANG, Mingqian, Quzhou, Zhejiang 324302 (CN); TANG, Yanjun, Quzhou, Zhejiang 324302 (CN); XU, Weidong, Quzhou, Zhejiang 324302 (CN); WU, Qiang, Quzhou, Zhejiang 324302 (CN); QIN, Shufang, Quzhou, Zhejiang 324302 (CN)
(74) Representative: Dai, Simin
(86) International application number: PCT/CN2024/088996
(87) International publication number: WO 2024/227405

(57) **Abstract**

A method for lignocellulose hydrolysis is provided. The method includes: crushing an agroforestry biomass feedstock to a particle size of 60-80 mesh, extracting with a toluene-ethanol solution, and drying at a constant temperature to obtain a de-extracted feedstock; weighing a metal salt and glycerol with a molar ratio of 1:10-1:124 for mixing at a temperature of 80 ° C - 90 ° C and a stirring speed of 180 rpm to obtain a type IV deep eutectic solvent which is homogeneous and transparent; weighing the de-extracted feedstock and adding to the type IV deep eutectic solvent prepared, and reacting for 2-4 h at a temperature of 80 ° C - 120 ° C and a stirring speed of 300 rpm-500 rpm to obtain a pretreatment mixture; performing a solid-liquid separation to the pretreatment mixture to obtain a filter residue and a filtrate, and then drying the filter residue after washing to obtain a lignocellulose residue after pretreatment; and performing enzymatic hydrolysis reaction to the lignocellulose residue to obtain fermentable sugar.

## Description

### TECHNICAL FIELD

The present disclosure generally relates to lignocellulose, and particularly relates to a method for lignocellulose hydrolysis.

### BACKGROUND

Lignocellulose is mainly composed of cellulose, hemicellulose, and lignin, and the efficient saccharification of cellulose is the key to the production of chemicals and liquid fuels. To realize the efficient conversion of the lignocellulose, it is necessary to provide an efficient and green pretreatment solvent system to destroy the anti-degradation barrier of the lignocellulose.

The pretreatment solvent system can effectively break an original compact structure of biomass and reduce the anti-degradation barrier, thereby facilitating the conversion of carbohydrates into fermentable monosaccharides or fuel ethanol. The green, mild, and efficient pretreatment solvents are of great significance for the enzymatic saccharification of lignocellulose.

As a new type of ionic liquid, deep eutectic solvents (DES) are formed by simple physical mixing of hydrogen bond donors and hydrogen bond acceptors, which can effectively remove lignin or hemicellulose, have little effect on cellulose degradation, and can also recycle the solvent.

### SUMMARY

According to an aspect of the present disclosure, a method for lignocellulose hydrolysis is provided by using an inexpensive and green low eutectic solvent system for enzymatic hydrolysis of lignocellulose. The method has the advantages of low equipment requirements, simple operation, effective improvement of enzymatic hydrolysis efficiency, etc.

Embodiments of the present disclosure provide a method for lignocellulose hydrolysis. The method includes:
step 1, crushing an agroforestry biomass feedstock to a particle size of 60-80 mesh, extracting with a toluene-ethanol solution, and drying at a constant temperature to obtain a de-extracted feedstock;
step 2, weighing a metal salt and glycerol with a molar ratio of 1:10-1:124 for mixing at a temperature of 80 ° C - 90 ° C and a stirring speed of 180 rpm to obtain a type IV deep eutectic solvent which is homogeneous and transparent, wherein the metal salt is one of aluminum chloride, zinc chloride, or ferric chloride;
step 3, weighing the de-extracted feedstock with a mass ratio of 1:5-1:15 and adding to the type IV deep eutectic solvent, and reacting for 2-4 h at a temperature of 80 ° C - 120 ° C and a stirring speed of 300 rpm-500 rpm to obtain a pretreatment mixture;
step 4, performing a solid-liquid separation on the pretreatment mixture using a centrifugal separation or a reduced-pressure filtration to obtain a filter residue and a filtrate, washing and desalting the filter residue with ethanol or deionized water until no metal salt is detected in a wash solution, and then drying the filter residue after washing to obtain a lignocellulose residue after pretreatment of the agroforestry biomass feedstock; and
step 5, adding the lignocellulose residue after pretreatment to an acetic acid buffer solution with a pH of 4.8 at a predetermined substrate concentration, adding cellulase complex, and reacting in an air-bath shaker at a temperature of 45 °C~50 °C and a stirring speed of 140 rpm ~ 150 rpm for a period of time to obtain fermentable sugar.

Currently, DES are categorized into four types, and a solvent system prepared using a metal salt and glycerol in the present disclosure is a type IV deep eutectic solvent system. Glycerol is an organic solvent commonly used in pretreatment, and when used alone in pretreatment, it requires a higher temperature and a longer period of time to achieve lignin removal, and in an acidic environment, it can improve removal, and the Lewis acidity provided by the metal salt can improve the lignin removal ratio, effectively degrade hemicellulose, and have a swelling rather than a dissolving effect on cellulose. The type IV deep eutectic solvent system of the present disclosure has high stability, is easy to prepare, and is a more promising green solvent for application.

In some embodiments, in step 1, agroforestry biomass of the agroforestry biomass feedstock is bamboo.

In some embodiments, in step 4, a drying condition of the filter residue after washing is drying at 80 °C until constant weight.

In some embodiments, in step 5, the predetermined substrate concentration of the lignocellulose residue after pretreatment is in a range of 2 wt%-10 wt%, the cellulase complex is Novozymes Cellic^{®} CTec2, an addition amount of the cellulase complex is 15 FPU/g of substrate-50 FPU/g of substrate, and a reaction time of enzymatic hydrolysis reaction is 3 h-48 h.

Compared with the prior art, the method for lignocellulose hydrolysis provided in the present disclosure employs the type IV deep eutectic solvent prepared by metal salt-glycerol to pretreat lignocellulose in the agroforestry biomass feedstock, and then performs an enzymatic hydrolysis reaction to the lignocellulose residue after pretreatment, having advantages of low cost, easy preparation, mild treatment conditions and short treatment time, as well as low requirements for equipment, easy control of the reaction and green and clean process. On the other hand, the cellulose looseness of the lignocellulose residue after pretreatment is improved, and a large amount of hemicellulose and lignin is removed, which is conducive to promoting the contact between the cellulase enzyme and the substrate and thereby improving the efficiency of cellulase hydrolysis.

### BRIEF DESCRIPTION OF THE DRAWINGS

FIG. 1 is a flowchart illustrating an exemplary method for lignocellulose hydrolysis according to some embodiments of the present disclosure;
FIG. 2 shows scanning electron micrographs of bamboo powder before pretreatment (a1, a2, a3) and after pretreatment (b1, b2, b3) by a type IV deep eutectic solvent prepared by ferric chloride-glycerol;
FIG. 3 is a comparison schematic diagram of an enzymatic hydrolysis efficiency (i.e., glucose conversion) of Example 1-Example 6 and Comparison Example of the present disclosure; and
FIG. 4 is a comparison schematic diagram of a hemicellulose removal ratio and a lignin removal ratio of a moso bamboo residue after pretreatment of Example 1-Example 6 of the present disclosure.

### DETAILED DESCRIPTION

In order to make the technical problems to be solved, technical solutions, and beneficial effects in the present disclosure clearer and more understandable, the following disclosure is described in further detail in combination with the accompanying drawings and embodiments. It should be understood that the specific embodiments described herein are only for explaining the present disclosure, and are not intended to limit the present disclosure.

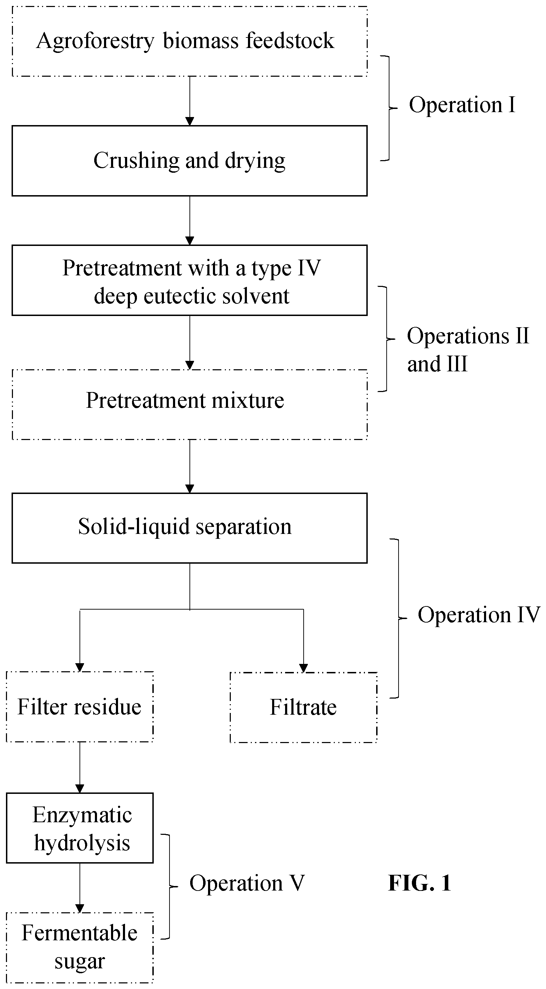

Referring to FIG. 1, the present disclosure also provides a preferred embodiment of a method for lignocellulose hydrolysis, including the following operations.

In Operation I, an agroforestry biomass feedstock is crushed to a particle size of 60-80 mesh, extracted with a toluene-ethanol solution with a volume ratio of 2:1, and dried at a constant temperature of 80 °C to obtain a de-extracted feedstock. Agroforestry biomass of the agroforestry biomass feedstock is moso bamboo.

In Operation II, a metal salt and glycerol are weighed and mixed at a molar ratio of 1:10-1:124, and the mixture is stirred at a stirring speed of 180 rpm and a temperature of 80-90 °C to obtain a type IV deep eutectic solvent which is homogeneous and transparent. The metal salt is one of aluminum chloride, zinc chloride, or iron chloride. In some embodiments, the molar ratio of the metal salt to glycerol may be 1:124. In some embodiments, according to the final pretreatment result, the metal salt may be ferric chloride.

In Operation III, the de-extracted feedstock prepared in Operation I is weighed and added to the type IV deep eutectic solvent prepared in Operation II at a mass ratio of 1:5-1:15, and reacts at a temperature of 80-120 °C with a stirring speed of 300-500 rpm for 2 h-4 h to obtain a pretreatment mixture. In some embodiments, the mass ratio may be 1:10.

In Operation IV, a solid-liquid separation is performed to the pretreatment mixture through centrifugal separation or reduced-pressure filtration, a filter residue and a filtrate are obtained respectively, the filter residue is washed and desalted with ethanol or deionized water until no metal salt component is detected in the wash solution, and the filter residue after washing is then dried to obtain a lignocellulose residue after pretreatment of the agroforestry biomass feedstock, and a drying condition of the filter residue after washing is drying at 80 °C until constant weight.

In Operation V, the lignocellulose residue after pretreatment is added to an acetic acid buffer solution with a pH of 4.8 at a certain substrate concentration, and then cellulase complex is added, and a reaction is carried out in an air-bath shaker at a temperature of 45-50 °C and a stirring speed of 140-150 rpm for a period of time to obtain fermentable sugar.

The substrate concentration of the lignocellulose residue after pretreatment is in a range of 2 wt%-10 wt%. The cellulase complex is Novozymes Cellic^{®} CTec2. An addition amount of the cellulase complex is in a range of 15 FPU/g substrate-50 FPU/g substrate. The enzymatic hydrolysis reaction time is in a range of 3 h-48 h. In some embodiments, the substrate concentration of the lignocellulose residue after pretreatment may be 2 wt%. The greater the substrate concentration, the longer it takes for the cellulase hydrolysis to reach a plateau. The cellulase complex is Novozymes Cellic@CTec2, different addition amounts of the cellulase complex have different effects on the effect and the required time of enzyme hydrolysis, and affect the experiments and the future cost of production. In order to achieve high enzyme hydrolysis efficiency while reducing the production cost, a preferred addition amount of the cellulase complex is 15 FPU/g substrate, the enzyme hydrolysis reaction time is in a range of 3 h-48 h. A preferred hydrolysis reaction time is 48 h according to the lignocellulose residue after pretreatment, the substrate concentration, the addition amount of the cellulase complex, optimization of the substrate enzyme hydrolysis effect, and saving of the enzyme hydrolysis time.

The type IV deep eutectic solvent prepared using the metal salt-glycerol in the present disclosure has the characteristics of being inexpensive and easily available, recyclable during utilization, and green and environmentally friendly. Moreover, the metal salt-glycerol has a very good effect on the removal of hemicellulose and lignin and has very little solubilization on cellulose, which leads to the high conversion efficiency of the cellulose.

The method for improving the efficiency of lignocellulose hydrolysis of the present disclosure is further described below through Examples and Comparison Example.

### Example 1

The present disclosure provides a first embodiment of the method for lignocellulose hydrolysis, including the following operations.

The moso bamboo feedstock was crushed and sieved to 60-80 mesh, and then extracted by a toluene-ethanol solution with a volume ratio of 2:1, and then dried at a constant temperature of 80 °C until constant weight, and a de-extracted feedstock was obtained. Ferrous chloride and glycerol were weighed in a molar ratio of 1:10 and stirred at 180 rpm until a homogeneous and transparent liquid was obtained. The de-extracted feedstock was weighed and added to the ferric chloride-glycerol solvent in a mass ratio of 1:15 and reacted for 4 h at a temperature of 80 ° C and a stirring speed of 300 rpm. At the end of the reaction, a solid-liquid separation was performed by vacuum filtration, and a separated moso bamboo filter residue was washed and desalted with deionized water until no metal salt was detected in a wash solution, to obtain a moso bamboo residue pretreated with the type IV deep eutectic solvent prepared by ferric chloride-glycerol. The moso bamboo residue was added to an acetate buffer solution (also referred to as an acetic acid buffer solution) at pH 4.8 and 5% substrate concentration, cellulase complex (Cellic^{®}CTec2) with 50 FPU/g substrate of the moso bamboo residue was added, and the reaction was carried out for 48 h at 50 °C in an air-bath shaker at 140 rpm to obtain glucose, which is fermentable sugar. Finally, the enzymatic hydrolysis efficiency of the pretreated moso bamboo residue was measured to be 46.63%.

### Comparison Example

The moso bamboo feedstock was directly subjected to enzyme hydrolysis without pretreatment with the type IV deep eutectic solvent prepared by metal salt-glycerol, and the efficiency of the moso bamboo in directly subjected to enzyme hydrolysis was analyzed. The Comparison Example includes the following operations.

The moso bamboo feedstock was crushed and sieved to 60-80 mesh, and then extracted by a toluene-ethanol solution, and then dried at 80 °C until constant weight, and a de-extracted feedstock was obtained. The de-extracted feedstock of the moso bamboo was added to acetate buffer solution at pH 4.8 and 2% substrate concentration, cellulase complex (Cellic^{®}CTec2) with 15 FPU/g substrate of the de-extracted feedstock was added, and the reaction was carried out for 48 h at 50 °C in an air-bath shaker at 140 rpm. The enzyme hydrolysis efficiency was 17.78%.

The enzymatic hydrolysis efficiency of Example 1 is 2.6 times more than the enzymatic hydrolysis efficiency of the Comparison Example, indicating that the DES pretreatment process of the present disclosure significantly improves the enzymatic hydrolysis efficiency of moso bamboo.

### Example 2

The present disclosure provides a second embodiment of the method for lignocellulose hydrolysis, including the following operations.

The moso bamboo feedstock was crushed and sieved to 60-80 mesh, and then extracted by a toluene-ethanol solution with a volume ratio of 2:1, and then dried at a constant temperature 80 °C until constant weight, and a de-extracted feedstock was obtained. Ferrous chloride and glycerol were weighed in a molar ratio of 1:124 and stirred at 180 rpm until a homogeneous and transparent liquid was obtained. The de-extracted feedstock was weighed and added to the ferric chloride-glycerol solvent in a mass ratio of 1:10, and reacted for 3 h at a temperature of 100 ° C and a stirring speed of 300 rpm. At the end of the reaction, a solid-liquid separation was performed by vacuum filtration, and a separated moso bamboo filter residue was washed and desalted with deionized water until no metal salt was detected in a wash solution, to obtain a moso bamboo residue pretreated with the type IV deep eutectic solvent prepared by ferric chloride-glycerol. The moso bamboo residue was added to an acetate buffer solution at pH 4.8 and at 2 % substrate concentration, cellulase complex (Cellic^{®}CTec2) with 15 FPU/g substrate of the moso bamboo residue was added, and the reaction was carried out for 48 h at 50 °C in an air-bath shaker at 140 rpm to obtain glucose, which is fermentable sugar. The enzymatic hydrolysis efficiency of the pretreated moso bamboo residue was measured to be 67.87%, which is 3.8 times more than the enzymatic hydrolysis efficiency of the moso bamboo without pretreatment of the Comparison Example.

### Example 3

The present disclosure provides a third embodiment of the method for lignocellulose hydrolysis, including the following operations.

The moso bamboo feedstock was crushed and sieved to 60-80 mesh, and then extracted by a toluene-ethanol solution with a volume ratio of 2:1, and then dried at a constant temperature 80 °C until constant weight, and a de-extracted feedstock was obtained. Ferrous chloride and glycerol were weighed in a molar ratio of 1:124 and stirred at 180 rpm until a homogeneous and transparent liquid was obtained. The de-extracted feedstock was weighed and added to the ferric chloride-glycerol solvent in a mass ratio of 1:10, and reacted for 2 h at a temperature of 120 ° C and a stirring speed of 300 rpm. At the end of the reaction, a solid-liquid separation was performed by vacuum filtration, and a separated moso bamboo filter residue was washed and desalted with deionized water until no metal salt was detected in a wash solution, to obtain a moso bamboo residue pretreated with the type IV deep eutectic solvent prepared by ferric chloride-glycerol. The moso bamboo residue was added to an acetate buffer solution at pH 4.8 and at 2 % substrate concentration, cellulase complex (Cellic^{®}CTec2) with 15 FPU/g substrate of the moso bamboo residue was added, and the reaction was carried out for 48 h at 50 °C in an air-bath shaker at 140 rpm to obtain glucose, which is fermentable sugar. The enzymatic hydrolysis efficiency of the pretreated moso bamboo residue was measured to be 91.13 %, which is 5.1 times more than the enzymatic hydrolysis efficiency of the moso bamboo without pretreatment of the Comparison Example.

### Example 4

The present disclosure provides a fourth embodiment of the method for lignocellulose hydrolysis, including the following operations.

The moso bamboo feedstock was crushed and sieved to 60-80 mesh, and then extracted by a toluene-ethanol solution with a volume ratio of 2:1, and then dried at a constant temperature 80 °C until constant weight, and a de-extracted feedstock was obtained. Ferrous chloride and glycerol were weighed in a molar ratio of 1:15 and stirred at 180 rpm until a homogeneous and transparent liquid was obtained. The de-extracted feedstock was weighed and added to the ferric chloride-glycerol solvent in a mass ratio of 1:5, and reacted for 3 h at a temperature of 100 ° C and a stirring speed of 300 rpm. At the end of the reaction, a solid-liquid separation was performed by vacuum filtration, and a separated moso bamboo filter residue was washed and desalted with deionized water until no metal salt was detected in a wash solution, to obtain a moso bamboo residue pretreated with the type IV deep eutectic solvent prepared by ferric chloride-glycerol. The moso bamboo residue was added to an acetate buffer solution at pH 4.8 and at 10 % substrate concentration, cellulase complex (Cellic^{®}CTec2) with 50 FPU/g substrate of the moso bamboo residue was added, and the reaction was carried out for 48 h at 50 °C in an air-bath shaker at 140 rpm to obtain glucose, which is fermentable sugar. The enzymatic hydrolysis efficiency of the pretreated moso bamboo residue was measured to be 69.41%, which is 3.9 times more than the enzymatic hydrolysis efficiency of the moso bamboo without pretreatment of the Comparison Example.

### Example 5

The present disclosure provides a fifth embodiment of the method for lignocellulose hydrolysis, including the following operations.

The moso bamboo feedstock was crushed and sieved to 60-80 mesh, and then extracted by a toluene-ethanol solution with a volume ratio of 2:1, and then dried at a constant temperature 80 °C until constant weight, and a de-extracted feedstock was obtained. Zinc chloride and glycerol were weighed in a molar ratio of 1:124 and stirred at 180 rpm until a homogeneous and transparent liquid was obtained. The de-extracted feedstock was weighed and added to the zinc chloride-glycerol solvent in a mass ratio of 1:10, and reacted for 3 h at a temperature of 100 ° C and a stirring speed of 300 rpm. At the end of the reaction, a solid-liquid separation was performed by vacuum filtration, and a separated moso bamboo filter residue was washed and desalted with deionized water until no metal salt was detected in a wash solution, to obtain a moso bamboo residue pretreated with the type IV deep eutectic solvent prepared by zinc chloride-glycerol. The moso bamboo residue was added to an acetate buffer solution at pH 4.8 and at 2 % substrate concentration, cellulase complex (Cellic^{®}CTec2) with 15 FPU/g substrate of the moso bamboo residue was added, and the reaction was carried out for 48 h at 50 °C in an air-bath shaker at 140 rpm to obtain glucose, which is fermentable sugar. The enzymatic hydrolysis efficiency of the pretreated moso bamboo residue was measured to be 24.75%, which is 1.4 times more than the enzymatic hydrolysis efficiency of the moso bamboo without pretreatment of the Comparison Example.

### Example 6

The present disclosure provides embodiments of a sixth embodiment of the method for lignocellulose hydrolysis, including the following operations.

The moso bamboo feedstock was crushed and sieved to 60-80 mesh, and then extracted by a toluene-ethanol solution with a volume ratio of 2:1, and then dried at a constant temperature 80 °C until constant weight, and a de-extracted feedstock was obtained. Aluminum chloride and glycerol were weighed in a molar ratio of 1:124 and stirred at 180 rpm until a homogeneous and transparent liquid was obtained. The de-extracted feedstock was weighed and added to the aluminum chloride-glycerol solvent in a mass ratio of 1: 10, and reacted for 3 h at a temperature of 100 ° C and a stirring speed of 300 rpm. At the end of the reaction, a solid-liquid separation was performed by vacuum filtration, and a separated moso bamboo filter residue was washed and desalted with deionized water until no metal salt was detected in a wash solution, to obtain a moso bamboo residue pretreated with the type IV deep eutectic solvent prepared by aluminum chloride-glycerol. The moso bamboo residue was added to an acetate buffer solution at pH 4.8 and at 2 % substrate concentration, cellulase complex (Cellic^{®}CTec2) with 15 FPU/g substrate of the moso bamboo residue was added, and the reaction was carried out for 48 h at 50 °C in an air-bath shaker at 140 rpm to obtain glucose, which is fermentable sugar. The enzymatic hydrolysis efficiency of the pretreated moso bamboo residue was measured to be 59.78 %, which is 3.4 times more than the enzymatic hydrolysis efficiency of the moso bamboo without pretreatment of the Comparison Example.

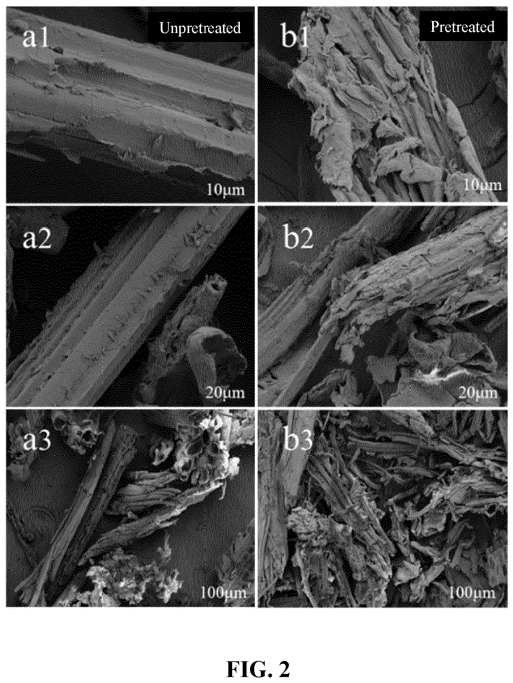

As shown in FIG. 2, a surface of bamboo fibers of unpretreated bamboo powder is relatively smooth and intact, for example, as shown in FIG. 2-a1, FIG. 2-a2, and FIG. 2-a3, the bamboo fibers are tightly aligned, while a surface of bamboo fibers of bamboo powder pretreated with the type IV deep eutectic solvent prepared by ferric chloride- glycerol of the present disclosure is rough and has deepening grooves, for example, as shown in FIG. 2 -b1, FIG. 2 -b2, and FIG. 2 -b3, the fiber is broken in many places, the breakage degree of the fracture surface is high, which greatly increased a contact area between the cellulase and the cellulose and an adsorption capacity of the cellulose to the cellulase, thereby improving the efficiency of the enzyme hydrolysis of the cellulose.

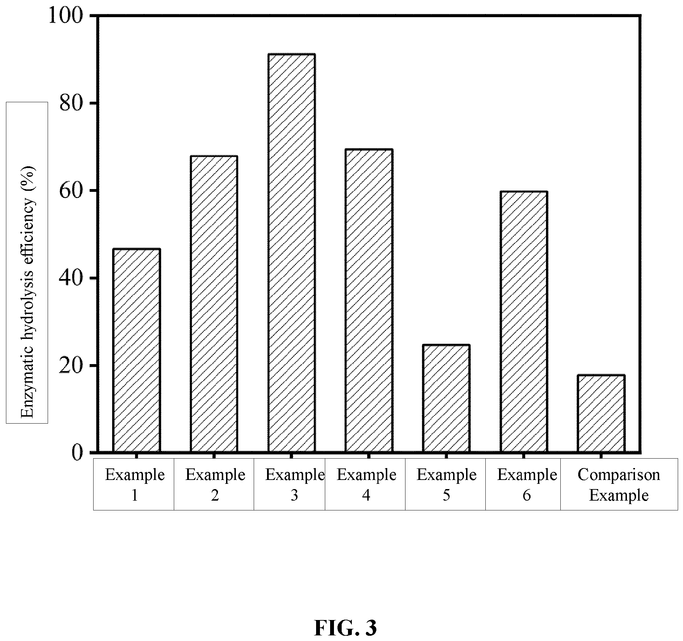

As shown in FIG. 3, compared with the Comparison Example, the enzymatic hydrolysis efficiency of the lignocellulosic biomass was improved after being pretreated by the type IV deep eutectic solvent synthesized from metal salt-glycerol of the present disclosure, especially the ferric chloride-based DES and alumina chloride-based DES. The enzymatic hydrolysis is closely related to the nature of the metal salt, and the pretreatment process conditions had different influence degrees on the final enzymatic hydrolysis efficiency.

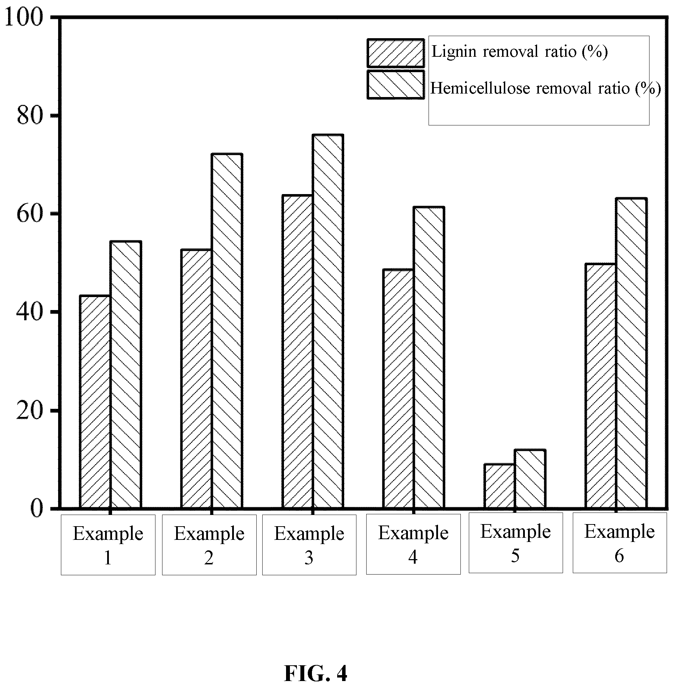

As shown in FIG. 4, the pretreatment process conditions and the pretreatment solvent systems have different influence degrees on the removal of the lignin and the hemicellulose. The removal ratio may, to some extent, reflect the destruction of the structural compactness of the lignocellulosic biomass. Therefore, lignin removal and hemicellulose removal can promote the enzymatic hydrolysis of lignocellulosic biomass and improve the efficiency of enzymatic hydrolysis.

The foregoing is only the preferred embodiments of the present disclosure and is not intended to limit the present disclosure, and any modifications, equivalent replacements, improvements, etc., made within the spirit and principles of the present disclosure shall be included in the scope of the present disclosure.

## Claims

1. A method for lignocellulose hydrolysis, comprising:
step 1, crushing an agroforestry biomass feedstock to a particle size of 60-80 mesh, extracting with a toluene-ethanol solution, and drying at a constant temperature to obtain a de-extracted feedstock;
step 2, weighing a metal salt and glycerol with a molar ratio of 1:10-1:124 for mixing at a temperature of 80 ° C - 90 ° C and a stirring speed of 180 rpm to obtain a type IV deep eutectic solvent which is homogeneous and transparent, wherein the metal salt is one of aluminum chloride, zinc chloride, or ferric chloride;
step 3, weighing the de-extracted feedstock prepared in step 1 with a mass ratio of 1:5-1:15 and adding to the type IV deep eutectic solvent prepared in step 2, and reacting for 2-4 h at a temperature of 80 ° C - 120 ° C and a stirring speed of 300 rpm-500 rpm to obtain a pretreatment mixture;
step 4, performing a solid-liquid separation on the pretreatment mixture using a centrifugal separation or a reduced-pressure filtration to obtain a filter residue and a filtrate, washing and desalting the filter residue with ethanol or deionized water until no metal salt is detected in a wash solution, and then drying the filter residue after washing to obtain a lignocellulose residue after pretreatment of the agroforestry biomass feedstock; and
step 5, adding the lignocellulose residue after pretreatment to an acetic acid buffer solution with a pH of 4.8 at a predetermined substrate concentration, adding cellulase complex, and reacting in an air-bath shaker at a temperature of 45 °C~50 °C and a stirring speed of 140 rpm ~ 150 rpm for a period of time to obtain fermentable sugar.

2. The method according to claim 1, wherein in step 1, agroforestry biomass of the agroforestry biomass feedstock is bamboo.

3. The method according to claim 1, wherein in step 4, a drying condition of the filter residue after washing is drying at a temperature of 80 °C until constant weight.

4. The method according to claim 1, wherein in step 5, the predetermined substrate concentration of the lignocellulose residue after pretreatment is in a range of 2 wt% to 10 wt%, the cellulase complex is Novozymes Cellic^{®}CTec2, an addition amount of the cellulase complex is in a range of 15 FPU/g of substrate - 50 FPU/g of substrate, and a reaction time of an enzymatic hydrolysis reaction is in a range of 3 h~48 h.
